(19) Europäisches Patentamt
European Patent Office
Office européen des brevets

(11) Publication number: 0 258 666 A2

(12) **EUROPEAN PATENT APPLICATION**

(21) Application number: 87111278.5

(22) Date of filing: 04.08.87

(51) Int. Cl.4: C12P 7/40 , C12P 41/00 , C12N 11/00 , C12N 15/00 , //(C12P7/40,C12R1:66)

(30) Priority: 11.08.86 US 895050
24.06.87 US 65965

(43) Date of publication of application: 09.03.88 Bulletin 88/10

(84) Designated Contracting States: AT BE CH DE ES FR GB GR IT LI LU NL SE

(71) Applicant: The Board of Regents of The University of Texas System
Office of General Counsel The University of Texas System 201 West Seventh Street
Austin Texas 78701(US)

(72) Inventor: Davis, Patrick J.
4704 Fieldstone drive
Austin Texas 78735(US)
Inventor: Miski, Mahmud
1020 East 45th Street, Apt. 121
Austin Texas 79751(US)

(74) Representative: Strehl, Schübel-Hopf, Groening, Schulz
Widenmayerstrasse 17 Postfach 22 03 45
D-8000 München 22(DE)

(54) **Microbial enzymatic transformations of chrysanthemol, lavandulol and analogous alcohols to acids.**

(57) A method for the production of chrysanthemic, lavandulic and analogous acids involving adding together and incubating under oxidizing conditions the corresponding chrysanthemol, lavandulol or analogous alcohol substrate and one or more oxidizing enzymes originated from a microorganism.

EP 0 258 666 A2

# MICROBIAL ENZYMATIC TRANSFORMATIONS OF CRYSANTHEMOL, LAVANDULOL AND ANALOGOUS ALCOHOLS TO ACIDS

## BACKGROUND OF THE INVENTION

### 1. Field of the Invention

This invention relates to the microbial synthesis of certain stereoisomers of chrysanthemic acid and analogous acids from chrysanthemol and analogous alcohols. The alcohol substrate is transformed stereoselectively to the acid by oxidizing enzymes originating from microorganisms.

### 2. Description of the Prior Art.

The constituents of some chrysanthemum species of plants have been used as insecticides since the early 19th century. See U.S. Patent No. 4,525,455 which is incorporated herein in its entirety by reference. The active constituents, e.g. pyrethrin I and pyrethrin II, are the esters of (1) the alcohols pyrethrolone, cinerolone or jasmolone and (2) the acids chrysanthemic acid (2,2-dimethyl-3-(2-methyl-1-propenyl)-1-cyclopropanecarboxylic acid) and pyrethric acid (3-(2-methoxycarbonyl-1-propenyl)-2,2-dimethyl-1-cyclopropanecarboxylic acid) respectively.

Synthetic analogues of the natural chrysanthemic acid esters have recently been developed which are remarkable for their extraordinary insecticidal activity and photostability. These synthetic analogues, designated pyrethroids, derive from cyclopropanecarboxylic acids in which the three membered ring contains two geminal methyl groups and a B-substituted vinyl group. Important pyrethroid acids include, in addition to chrysanthemic acid (X = Y = $-CH_3$) and pyrethric acid (X = $-COOCH_3$, Y = $-CH_3$), dichlorochrysanthemic acid (X = Y = -Cl) and dibromochrysanthemic acid (X = Y = -Br) which are shown by the formula

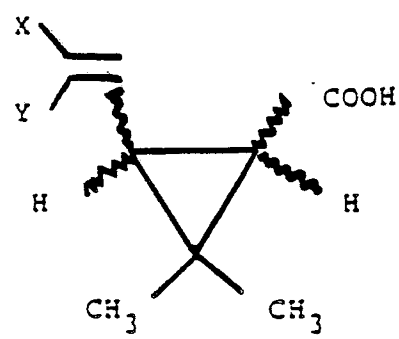

The superior activity of trans-chrysanthemic esters, and especially of the stereoisomer having the (1R, 3R) (+)-trans configuration has long been known. For example, the greatest insecticidal activity is found with the (1R,3R) (+)-trans isomer of the chrysanthemic ester in combination with the natural pyrethroid alcohol components pyrethrolone, cinerolone and jasmolone and in synthetic chrysanthemates such as bioallethrin (alcohol = cis or trans-allethrolene) and tetramethrin (alcohol = neopynaminol). Similar relationships of activity to stereochemical structure have been found. As further examples, decamethrin (k-orthrin) contains as the acid component (1R,3R)-cis-3-(2,2-dibromovinyl)-2,2-dimethyl-1-cyclopropanecarboxylic acid (i.e. decamethrinic or dibromochrysanthemic acid). Still other stereoisomers of chrysanthemic acid or its analogues are preferred as the acid component.

Specific stereoisomers of lavandulic acid of the general formula

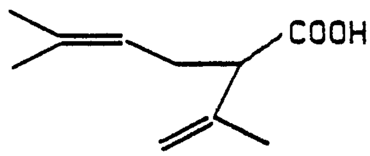

and its derivatives have also been shown to have insecticidal activity as reported by Elliott, et al., Pestic. Sci. 14:1-8(1983) and Ayad, et al., J. Agric. Food Chem. 32:85-92(1984), which is incorporated herein by reference.

In addition to their use as a component in insecticides, some of the pyrethroid acids and lavandulic acid derivatives in certain stereochemical configurations have broad industrial uses.

Because of the relationship of stereochemical structure to insecticidal activity or other industrial uses, major efforts have been made to develop methods of preparation of stereochemically uniform pyrethroid acids. Some of these efforts are summarized in D. Arlt, et al., ANGEWANDTE CHEMIE - International English Edition, Vol. 20, No. 9, pages 703-818 (September 1981), which is incorporated herein in its entirety by reference. Notwithstanding these efforts, however, there is an acutely felt need for cost effective stereoselective and anantioselective methods of synthesis of pyrethroid acids and lavandulic acid derivatives.

## SUMMARY OF THE INVENTION

This invention involves the discovery that certain microorganisms contain one or more enzymes which can transform stereoselectively chrysanthemol and analogous alcohols to their corresponding pyrethroid acids. Thus, naturally occurring plant metabolites such as, for example, (1R,3R) (+)-trans-chrysanthemol can be transformed to (1R,3R) (+)-trans-chrysanthemic acid with substantially all of that alcohol stereoisomer being transformed to the corresponding acid stereoisomer. This can be accomplished whether the (1R,3R) (+)-trans stereoisomer of the alcohol is contained in a mixture of other stereoisomers of the alcohol or whether the alcohol is stereochemically uniform, i.e. contains only that particular stereoisomer. Synthetically produced stereochemically uniform alcohols such as, for example, (1R,3R) (+)-cis-dibromochrysanthemol, or the same contained in a stereochemical mixture can be transformed to (1R,3R) (+)-cis-dibromochrysanthemic acid. Finally, a mixture of stereoisomers of an alcohol can be resolved to a particular stereoisomer or desired stereoisomers of the acid by use of enzyme(s) which selectively transform the desired stereoisomer(s) while leaving untransformed less desirable stereoisomers of the alcohol. The acid stereoisomer(s) resulting from the enzyme transformation can be separated from the untransformed alcohol stereoisomer(s) by known methods.

Accordingly, the invention involves a method for the microbial synthesis of an acid of the formula

I

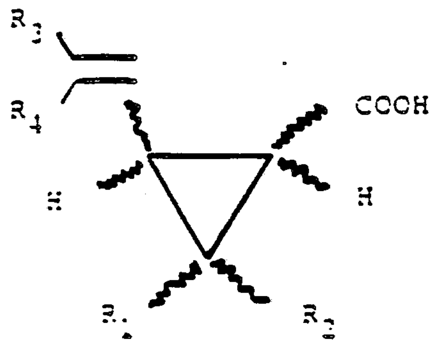

or

III

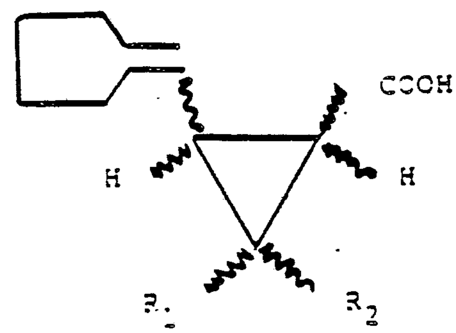

where an alcohol substrate of the formula respectively

II

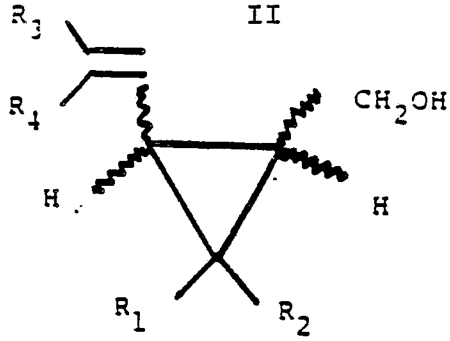

or

IV

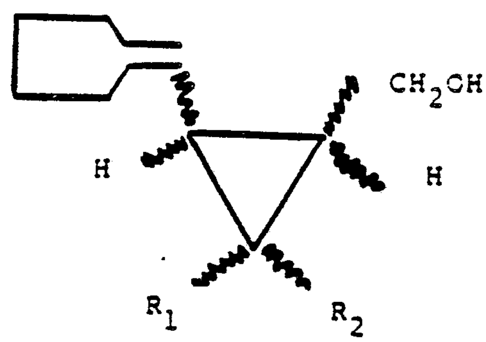

and one or more enzymes originated from a microorganism are added together and incubated under oxidizing conditions, wherein

$R_1$ is selected from the group consisting of a hydrogen atom, $-CH_3$, $-CH_2CH_3$, $-CH_2CH_2CH_3$, and a halogen atom;

$R_2$ is selected from the group consisting of a hydrogen atom, $-CH_3$, $-CH_2CH_3$, $-CH_2CH_2CH_3$, and a halogen atom;

and for the compounds shown on the left above

$R_3$ is selected from the group consisting of $-CH_3$, $-CH_2CH_3$, $-CH_2CH_2CH_3$, $-CH_2CH_2CH_2CH_3$, $-CH_2CH_2CH_2CH_2CH_3$, and a halogen atom; and

$R_4$ is selected from the group consisting of $-CH_3$, $CH_2CH_3$, $-CH_2CH_2CH_3$, $-CH_2CH_2CH_2CH_3$, $-CH_2CH_2CH_2CH_2CH_3$, and a halogen atom, and $COOCH_3$.

The transformation of alcohol to acid, catalysed by the enzyme(s), may be by a dehydrogenation reaction, oxidation reaction or otherwise.

The alcohol substrate may, for example, comprise all (a complete stereochemical mixture), one (a stereochemically uniform alcohol) or more (a partial stereochemical mixture) of the following stereoisomers

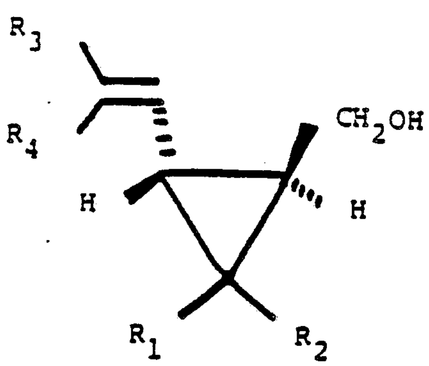

(1R,3R)(+)-<u>trans</u> stereoisomer

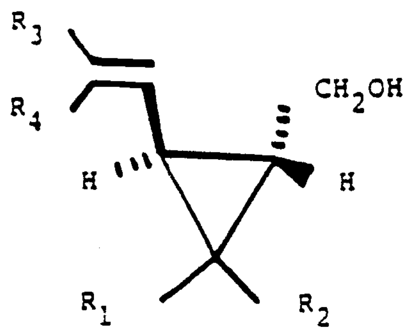

(1S,3S)(-)-trans stereoisomer

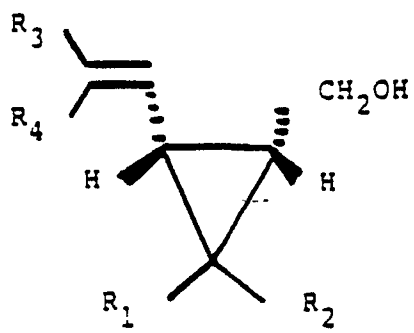

(1S,3R)(-)-cis stereoisomer

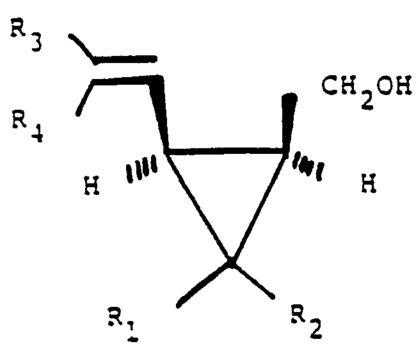

(1R,3S)(+)-cis stereoisomer

Depending upon which of the above stereoisomers are present in the alcohol substrate and of those present which will be transformed by the enzyme(s) to the stereochemically analogous acid, one or more of the following pyrethroid acids will be produced

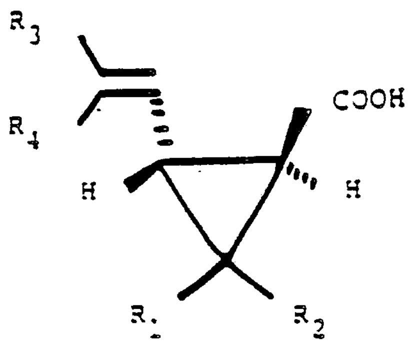

(1R,3R)(+)-trans stereoisomer

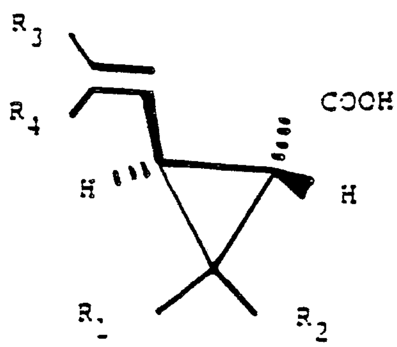

(1S,3S)(-)-trans stereoisomer

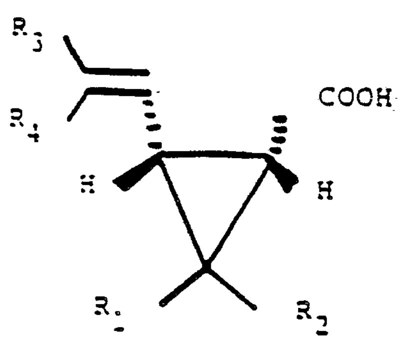

(1S,3R)(-)-cis stereoisomer

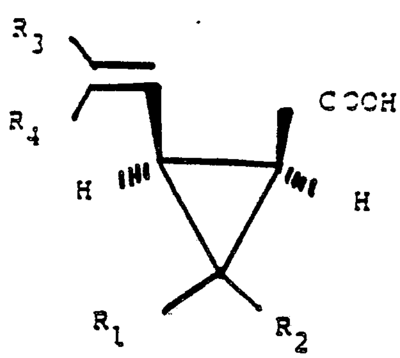

(1R,3S)(+)-cis stereoisomer

Note: The absolute configurations of the halogenated pyrethroid alcohols and acids are as shown with each structural formula above. However, according to convention, the designation of absolute configuration (i.e., R vs S) is inverted at $C_1$ if $R_1$ and/or $R_2$ are halogens, and is inverted at $C_3$ if $R_3$ and/or $R_4$ are halogens. For example, (+) (trans)-chrysanthemic acid where $R_1 = R_2 = R_3 = R_4 = CH_3$ is designated 1R, 3R, whereas the (+) trans-acid where $R_1 = R_2 =$ halogen, $R_3 = R_4 = CH_3$ is designated 1S, 3R. Likewise, the (+) trans-acid where $R_1 = R_2 = CH_3$, $R_3 = R_4 =$ halogens is designated 1R, 3S, even though all three of these compounds have the same absolute stereochemistry shown for (+) trans chrysanthemic acid.

This invention involves the discovery that lavandulol and analogous alcohols can also be stereoselectively transformed to their corresponding acids as described above for chrysanthemyl alcohols. Accordingly, the invention involves a method for the microbial synthesis of an acid of the formula

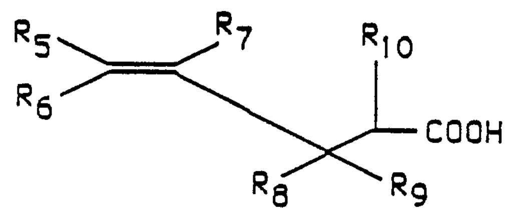

where an alcohol substrate of the formula

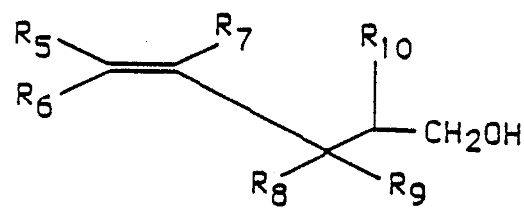

and one or more enzymes originated from a microorganism are added together and incubated under oxidizing conditions, wherein

$R_5$ is selected from the group consisting of $-CH_3$, a hydrogen atom and a halogen atom;
$R_6$ is selected from the group consisting of $-CH_3$, a hydrogen atom and a halogen atom;
$R_7$ is selected from the group consisting of $-CH_3$, a hydrogen atom and a halogen atom;
$R_8$ is selected from the group consisting of a hydrogen atom, $-CH_3$ and $-CH_2CH_3$;
$R_9$ is selected from the group consisting of a hydrogen atom and $-CH_3$; and
$R_{10}$ is selected from the group consisting of $-CH_3$, $-CH_2CH_3$, an isopropylene group, an isopropyl group, a sec-butyl group, an isobutyl group, a t-butyl group, a cyclopentyl group, a cyclopropyl group.

The transformation of alcohol to acid, catalyzed by the enzyme(s) may be dehydrogenation reaction, oxidation reaction or otherwise.

The alcohol substrate may, for example, comprise both (a complete stereochemical mixture) or one (a stereochemically uniform alcohol) of the following stereoisomers of lavandulol ($R_5 = R_6 = CH_3$, $R_7 = R_8 = R_9 =$ a hydrogen atom; $R_{10} =$ an isopropylene group)

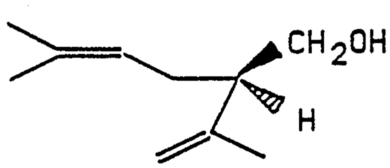

R-(-)-lavandulol

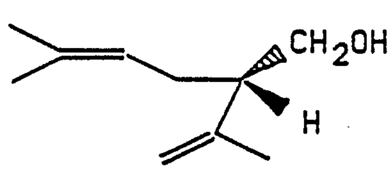

S-(+)-lavandulol

Depending upon which of the above stereoisomers are present in the alcohol substrate and of those present which will be transformed by the enzyme(s) to the stereochemically analogous acid, one or both of the

following acids will be produced

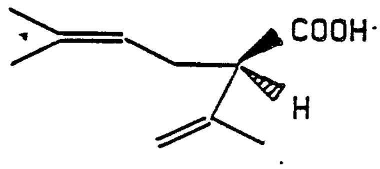

R-(-)-lavandulic acid

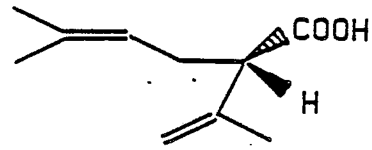

S-(+)-lavandulic acid

Note: References throughout this specification and the apended claims to R-(-) and S-(+) derivatives of lavandulol and lavandulic acid refer to the configuration around C-2 (i.e., the carbon to which $-CH_2OH$ and -COOH are attached, respectively). Although addition of various groups as $R_8$ and/or $R_9$ may create a second assymetric carbon center at C-3, such additions will not change the absolute configuration at C-2.

In general, where only some of the stereoisomers of the alcohol substrate are transformed to the stereochemically analogous stereoisomers of the pyrethroid of lavandulic acid in amounts in excess of trace amounts, the stereochemical mixture of the alcohols will in effect be resolved in the transformation to the acid. The acid stereoisomers may be separated from the residual untransformed alcohol stereoisomers by known means.

Within the Aspergillus genus, three species were found that transformed the alcohol substrate to the corresponding acid: Aspergillus ochraceus (ATCC 18500); Aspergillus flavipes (ATCC 1030); and Aspergillus flavipes (ATCC 11013). These microorganisms contain one or more enzymes which transform the alcohols to the corresponding acid, perhaps as a detoxification mechanism. These microorganisms contain genes which allow them to express these enzymes. Upon isolation of the genes coded for these enzymes, other microorganisms may be transformed by known means to express the same or substantially homologous enzymes.

The cell system of an active microorganism may be immobilized to provide a more stable, longer half-life bioreactor system that can be used in a continuous process. Cells may be immobilized in a variety of ways, including the covalent cross-linking of cells, covalent linkage to a rigid support, non-covalent (e.g. ionic) linkage to a support, non-covalent linkage by growth around an inert support, entrapment of the cells in a gel matrix, or microencapsulation. Entrapment in both synthetic (polyurethane, polyacrylamide) and natural (agar, carrageenan, cellulose) polymers represents the most popular method. Suspensions of cells are mixed with synthetic monomeric materials, and polymerization allowed to occur, or natural polymers are solubilized, mixed with cells, and then allowed to precipitate (coagulate) around the cells. The cells can then be packed into a number of laboratory bioreactor configurations, including packed-bed reactors, continuous-feed stirred tank reactors, fluidized bed reactors, or hollow fiber reactors. Transformations are affected by passage of a solution of the substrate over or through the catalyst (cell) bed of the bioreactor, and continuously harvesting product.

Alternatively, once enzymatic activity can be demonstrated by a microbial culture, a cell free system may be formed by disrupting the cells. The enzymes contained in the cell free system may be purified and isolated. Cell disruption can be affected by cell grinding with an abrasive, use of the Braun (or other ballistic) homogenizers, sonication, or by repeated freezing or thawing. See, for example, U.S. Patent No. 4,525,455, incorporated herein by reference. Initial crude purification may often be accomplished by ammonium sulfate fractionation. The active fraction is then purified by gel chromatography, electrophoresis, and/or by affinity chromatography. The pure enzyme (assuming it consists of a single component) would be expected to yield a constant specific activity (mmoles of substrate converted per mg protein per unit time) and a single band on electrophoresis. Ideally, the protein may be crystalline. the enzyme(s) may also be immobilized on a solid support.

## BRIEF DESCRIPTION OF THE FIGURES

FIG. 1 shows the gas chromatogram of the biotransformation of the stereoisomers of chrysanthemol by A. ochraceus (ATCC 18500).

FIG. 2 shows the gas chromatogram of the biotransformation of the stereoisomers of chrysanthemol by A. flavipes (ATCC 1030).

FIG. 3 is a graphic summary of the time-dependent biotransformation of the stereoisomers of chrysanthemol by A. ochraceus (ATCC 18500).

FIG. 4 is a graphic summary of the time-dependent biotransformation of the stereoisomers of chrysanthemol by A. flavipes (ATCC 1030).

FIG. 5 shows the gas chromatogram of the biotransformation of the stereoisomers of dibromochrysanthemol by A. ochraceus (ATCC 18500).

FIG. 6 shows the gas chromatogram of the biotransformation of the stereoisomers of dibromochrysanthemol by A. flavipes (ATCC 1030).

FIG. 7. shows the gas chromatogram of the biotransformation of the stereoisomers of dichlorochrysanthemol by A. ochraceus (ATCC 18500).

FIG. 8 shows the gas chromatogram of the biotransformation of the stereoisomers of dichlorochrysanthemol by A. flavipes (ATCC 1030).

## DESCRIPTION OF THE PREFERRED EMBODIMENT

1. Microbial Screening for Ability to Oxidize Chrysanthemols to Their Acids.

All cultures used in these studies were maintained on refrigerated (4°C) slants of Sabouraud-maltose agar (Difco Laboratories, Detroit, Mich.) and transferred every 6 months to maintain viability. The sources of cultures were as follows: ATCC, American Type Culture Collection, Rockville, Md.; NRRL, Northern Regional Research Laboratories, U.S. Department of Agricultural, Peoria, IL.; UI,J. P. Rosazza, College of Pharmacy, University of Iowa, Iowa City; CBS, Centraalbureau Voor Schimmelcultures, Baarn, Netherlands; NCYC, National Collection of Yeast Cultures, Brewing Industry Research Foundation, Nutfield, Surry, England. The fermentation media used in these studies consisted of the following compositions: A = dextrose, 20 g; soybean meal (20 mesh; Capital Feeds, Austin, TX.), 5 g; sodium chloride, 5g; potassium phosphate (dibasic), 5 g; yeast extract (Difco), 5 g; distilled water, 1,000 ml; pH adjusted to 7.0 with 6N HCl. B = dextrose, 20 g; acidicase peptone (BBL Microbiology Systems, Cockeysville, Md.), 5 g; potassium phophate (dibasic), 5 g; distilled and deionized water, 1,000 ml; pH adjusted to 7.0 with 6N HCl. Both media were sterilized in individual flasks at 121°C for 15 min. Incubations were conducted in 125 ml Bellco Delong culture flasks containing 25 ml of the medium, with an NBS model G-25R environmental shaker (New Brunswick Scientific Co., Inc., Edison, N.J.) operated at 250 rpm and 27°C.

For each culture examined, first-stage flasks were initiated by suspending surface growth or spores on agar slants in a portion of the medium contained in a flask and transferring the suspension to that 125 ml flask under aseptic conditions. After incubation for 72 hrs, a 2 ml portion of this first-stage culture was used to inoculate a second-stage flask of the same composition, and the incubation was allowed to continue for 24 h.

After 24 h in the second-stage, the substrates were added, and the incubation was allowed to continue. Samples (1 ml) were aseptically harvested. Samples were mixed with 1 ml Clark's buffer (25 ml of 0.2 M KCl plus 6.5 ml of 0.2 M HCl, diluted to 100 ml, pH 2.0) and extracted with 5 ml ether in capped tubes. The tubes were mixed for 30 min. at 19 rpm, and centrifuged (1500 × g) for 5 min. The organic layer was taken to dryness in the cold under vacuum and reconstituted with 0.2 ml ether. Samples were analyzed by gas chromatography after derivatisation with diazoethane as described below.

The microbial oxidation of (+/-)-cis/trans-chrysanthemols to the corresponding chrysanthemic acids by twenty-five different strains of fungi and yeasts shown in Table 1 were examined.

TABLE 1. Cultures screened for microbial oxidation of Chrysanthemols

| Culture | Medium |
| --- | --- |
| Aspergillus alliaceus Ul 315 | (A) |
| Aspergillus alliaceus NRRL 317/83 | (A) |
| Aspergillus flavipes ATCC 1030 | (A) |
| Aspergillus flavipes ATCC 11013 | (A) |
| Aspergillus flavipes ATCC 16795 | (A) |
| Aspergillus flavus ATCC 9170 | (A) |
| Aspergillus flavus ATCC 24714 | (A) |
| Aspergillus foetidus NRRL 337 | (A) |
| Aspergillus niger Ul-X-172 | (A) |
| Aspergillus niger NRRL 328/83 | (A) |
| Aspergillus niger ATCC 10548 | (A) |
| Aspergillus niger ATCC 10581 | (A) |
| Aspergillus niger ATCC 11394 | (A) |
| Aspergillus niger ATCC 16888 | (A) |
| Aspergillus ochraceus ATCC 18500 | (A) |
| Aspergillus ochraceus ATCC 22947 | (A) |
| Aspergillus parasiticus ATCC 15517 | (A) |
| Fusarium solani ATCC 12823 | (B) |
| Rhizopus arrhizus ATCC 11145 | (A) |
| Rhizopus arrhizus ATCC 20097/83 | (A) |
| Rhizopus stolonifer NRRL 1478/83 | (A) |
| Saccharomyces cerevisiae -wild type- | (A) |
| Saccharomyces cerevisiae NCYC 240 | (A) |
| Saccharomyces cerevisiae NRRL Y2034 | (A) |
| Saccharomyces lipolytica CBS 599 | (A) |

It was found that three Aspergilli transformed the stereoisomeric mixtures of chrysanthemols to chrysanthemic acids. Aspergillus flavipes ATCC 1030 and A. flavipes ATCC 11013 started this conversion 72 hours after substrate addition. Aspergillus ochraceus ATCC 18500 started the desired oxidation immediately after substrate addition. A. ochraceus (ATCC 18500) required 48 hrs for maximum oxidation, while the two A. flavipes strains required 96-120 hrs for maximum oxidation.

2. Gas Chromatographic Method Utilized in Screening Microbes.

Gas chromatographic analyses were conducted on a Shimadzu GC-4CM chromatograph with 5 m × 3 mm i.d. "silanized" glass column packed with 10% by wgt. QF-1 on Chromosorb GHP, 60/80 mesh (Foxboro-Analabs, Inc., North Haven, CT.). Flame ionization detectors were used, with nitrogen as the carrier gas. The following retention times (in minutes) were observed under the specified conditions of 185°C, carrier gas (CG) flow rate 14 ml/min racemic cis-chrysanthemols, 13.3 minutes; racemic t rans-chrysanthemols, 12.4 minutes; racemic cis-chrysanthemic acid ethyl esters, 15.5 minutes; racemic trans-chrysanthemic acid ethyl esters, 16.8 minutes; racemic cis-chrysanthemyl adehydes, 20.2 minutes; racemic trans-chrysanthemylaldehyde, 18.2 minutes.

3. Preparation of (+/-)-cis/trans -Chrysanthemols.

(+/-)-cis/trans -ethyl chrysanthemate (2 g) was dissolved in dry ether (60 ml), and lithium aluminum hydride (500 mg) added in small portions to the solution. Then, the mixture was refluxed for 4 hrs. Excess reagent was destroyed by dropwise addition of saturated ammonium chloride solution. After filtration of the precipitate, the solvent was evaporated under reduced pressure. A total of 1.45 g (+/-)-cis/trans-chrysanthemols was obtained without further isomerisation.

4. Derivatization of (+/-)-cis/trans-Chrysanthemic Acids with Diazoethane.

(+/-)-cis/trans-chrysanthemic acids (20 mg) were dissolved in 1 ml dry ether. Freshly prepared diazoethane (from 1-ethyl-3-nitro-1-nitrosoguanidine) in ether was added to this solution. Evaporation of the solvent 5 minutes later, yielded 21.5 mg of the ethyl chrysanthemate mixture (quantitative yield). The ethyl chrysanthemates generated by this method were used as standard materials for gas chromatographic analyses and to confirm metabolic results. The same method was used for derivatization of microbial extracts prior to GC analysis.

5. Gas Chromatographic Analysis of Individual Chrysanthemic Acid Stereoisomers Following Derivatization with R-(-)-2-octonol.

(+/-)-cis/trans-chrysanthemic acids (50 mg) were dissolved in 2 ml dry benzene. Freshly distilled thionyl chloride (78.4 mg) and pyridine (dry, 23.5 mg) were added subsequently. and stirred 3 hrs to form the corresponding acid chlorides according to the methods of M. Elliot, et al., Pestic. Sci., Vol. 11, p. 513 (1980) and C. Brookes, et al., Anal. Chem., Vol. 45, p. 896 (1973). Then R-(-)-2-octanol (38.7 mg) and pyridine (dry, 23.5 mg) were added to this solution and stirred for an additional 26 hrs at room temperature. After removal of solvent diastereomeric mixtures of R-(-)-2-octanol chrysanthemates were obtained as a yellow oil. A portion of this oil was subjected to GC analysis as described above. The retention time (in min.) of each diastereoisomer was:
(-)-cis-R-(-)-2-octanol chrysanthemate, 63.8;
(+)-cis-R-(-)-2-octanol chrysanthemate, 67.4;
(-)-trans-R-(-)-2-octanol chrysanthemate, 72.1; and
(+)-trans-R-(-)-2-octanol chrysanthemate, 76.9.

6. Medium Modification Experiments.

Initial screening experiments indicated that Aspergillus flavipes (ATCC 1030), A. flavipes (ATCC 11013) and A. ochraceus (ATCC 18500) were capable of transforming chrysanthemols to chrysanthemic acids. Studies were then undertaken to optimize these transformations and the following factors or components were changed or added at the stage 11 level:

1. Microcrystalline cellulose or cellite (5 g per liter) was added to the cultures to provide optimum growth by nucleation of pellet formation.

2. During transformations with A. flavipes (ATCC 1030) and A. ochraceus (ATCC 18500), the pH of cultures drifted towards a more basic pH (up to 8.4). Thus, the effect of initial pH adjustment was examined with A. flavipes (ATCC 1030), A. flavipes (ATCC 11013) and A. ochraceus (ATCC 18500). For this purpose five different initial pH values (4, 5, 6, 7 and 8) were used.

3. The effect of the presence of intense light during the transformation was examined.

4. The effect of the different sugar levels in the medium was also examined. Adjustments were over four different levels (in addition to normal media composition amount of 20g/l); a: 0.5, b: 1.5, c: 2 and d: 4 times of normal sugar amount. Each of these experiments was carried out in 125 ml (25 ml culture) second stage flasks. At a point in time 36 hr after inoculation, the substrate ((+/-)-trans/cis-chrysanthemols mixture, 65:35) (10 mg in 50 ul DMF) was added. Starting 24 hr after substrate addition, 2 ml culture aliquots were harvested at 24 hr intervals. Extraction, derivatization and GC analysis of each sample was done as described above.

Among the modifications examined, nucleation with microcrystalline cellulose showed very positive results with all cultures such as providing homogenous, thick growth with small pellet size and (probably in connection with better growth) reducing the required time for transformation ca. 1/3. Further, an initial pH 4 adjustment dramatically increased the transformation rate of A. flavipes (ATCC 1030), and the total conversion time was reduced from 96-120 hr to ca. 50 hr. Either increasing or reducing the sugar amount caused a delay in the transformation indicating that our initial level was optimal. The other modifications had little or no effect on the transformation.

7. Stereoselective Transformation of Chrysanthemols to Their Acids.

Eight-125 ml second stage flasks of A. ochraceus (ATCC 18500) and A. flavipes (ATCC 1030) were generated as described above. The substrate (+/-)-cis/trans-chrysanthemol was added at 24 hrs and the transformation course was followed by GC (185°C isothermal column temp, $N_2$ (CG) flow rate 14 ml min, extracted samples were derivatized with diazoethane). 96 hr after substrate addition each culture was harvested, acidified with an equal volume of the Clark's buffer, then extracted with ether. The solvent was removed carefully under reduced pressure in the cold and the residue was derivatized according to the method of A. Murano, Agr. Biol. Chem., Vol. 36, p. 2203 (1972): the residue was redissolved in 1 ml dry toluene, and 40 mg pyridine, 168 mg $SOCl_2$ and 460 mg R-(-)-2-octanol (each in 1 ml dry toluene) were added. This mixture was heated at 100° for 20 min, and the reaction then poured into water. This mixture was placed into a separatory funnel, the organic layer was washed with water, and then a 5% $NaHCO_3$ solution, and the organic layer was dried over anhydrous $MgSO_4$. The solvent was removed under reduced pressure in the cold. The residue was subjected to GC analysis as set forth above (5m × 3mm, 10% QF-1 on Chromosorb GHP, 60/80 mesh) with programmed temperature: a) 175 (initial)-210°C (1° per min), or b) 170-210° (2° per min). Retention times (in min) as follows:

(Programmed temperature condition specified in parenthesis)

(-)-R-2-octanyl-(-)-cis -chrysanthemate: 44.5. (a), 35.6 (b).

(-)-R-2-octanyl-(+)-cis-chrysanthemate: 46.5 (a), 37.7 (b).

(-)-R-2-octanyl-(-)-trans-chrysanthemate: 47.9 (a), 39.7 (b).

(-)-R-2-octanyl-(+)-trans-chrysanthemate: 50.0 (a), 41.7 (b).

GC analysis of the biotransformation results with A. ochraceus (ATCC 18500) clearly showed that this fungus was transforming (+)-cis-and (+)-trans-chrysanthemols to the corresponding (+)-cis-and (+)-trans-chrysanthemic acids with high stereoselectivity (see Figure 1). Only trace amounts, i.e. less than 10% by wgt., of the (-)-cis and (-)-trans of chrysanthemic acid were produced. Likewise, a similar stereoselectivity was observed for (+)-cis-chrysanthemol with A. flavipes (ATCC 1030) (see Figure 2). At 96 hrs, the (+)-trans and (-)-trans alcohol had already been transformed to the acid.

8. Preparative-Scale Oxidations of Chrysanthemols with Aspergillus Ochraceus.

In order to demonstrate the biotransformation stereoselectivity of A. ochraceus (ATCC 18500) by methods other than the chiral derivatization-GC procedure, each of the transformation products and residual substrates were isolated and fully characterized by their individual spectral properties.

The pure racemic mixtures of (+/-)-trans -chrysanthemol and (+/-)-cis-chrysanthemol were obtained by modifying the procedure of K. Ueda, et al., Tetrahedron, Vol. 27, p. 2771 (1971). 15 g (+/-)-cis/trans-chrysanthemic acid were obtained by alkaline hydrolysis of the commercially available ethyl ester mixture. This was dissolved in dry benzene (100 ml) and after addition of 1.5 ml $BF_3$-etherate complex, stirred overnight under a nitrogen atmosphere. The reaction was quenched by the addition of 20 ml of an ice-water mixture and stirring continued for 2 hr. The upper (benzene) layer was separated and dried over anhydrous $MgSO_4$. Evaporation of the solvent under reduced pressure yielded a 13.8 g mixture of (+/-)-cis-chrysanthemyl lactone (from the cis isomers present) and (+/-)-trans chrysanthemic acid. This mixture was reacted with excess of diazoethane in ether to convert the free (+/-)-trans-chrysanthemic acid to its ethyl esters. Following the esterification, the ether was removed under reduced pressure and the oily residue redissolved in hexane and extracted with a 5% aqueous NaOH solution. During this partition, the lactone ring opened, which then dissolved in the aqueous layer as its sodium salt. The hexane layer was separated and dried over anhydrous $MgSO_4$ and evaporated under reduced pressure to yield pure (+/-)-trans-ethylchrysanthemate (10.5 g) (purity ca. 99%). This compound was directly converted to (+/-)-trans-chrysanthemol by LAH reduction in dry ether as described above (yield 8.6 g).

Pure (+/-)-cis-chrysanthemic acid was obtained by fractional crystallization of 95% cis-enriched chrysanthemic acid mixtures from EtOAc and was then converted to (+/-)-cis-chrysanthemol by LAH reduction as described above.

Following the usual first stage, ten-liter second stage flasks (200 ml media) were used for each substrate transformation. At a point of time 36 hr after inoculation of the second stage flasks, 100 mg substrate (either (+/-)-cis or (+/-)-trans-chrysanthemol in 0.4 ml DMF) was added to each flask. Every 24 hr, 2 ml of culture was aseptically harvested to monitor the biotransformation by GC as described above, which indicated that 72 hr after substrate addition, the transformation was complete. The flask contents for each culture were harvested, combined, acidified with the addition of an equal volume of Clark's buffer and extracted with ether (3×300 ml).

The ether layer was dried over anhydrous $MgSO_4$ and carefully evaporated under reduced pressure in the cold. The oily residue was redissolved in hexane (200 ml) and extracted with 5% aqueous NaOH solution in a separatory funnel. This extraction allows separation of non-transformed chrysanthemols from transformed chrysanthemic acids (the latter extract into the basic aqueous layer as the sodium salts). The hexane layer was separated, and dried over anhydrous $MgSO_4$, and carefully evaporated in the cold under reduced pressure to yield the residual chrysanthemols. Following the acidification of the aqueous layer with 6N HCl, extraction was affected with ether, the ether layer separated and dried, and the solvent evaporated under reduced pressure in the cold to yield the chrysanthemic acid products. Both extracts were a greenish-yellow oily mixture.

Each of these mixtures were purified over a silica gel column (1.5×15 cm, 100-200 mesh), using 10% EtOAc in hexane as the mobile phase. The fractions containing chrysanthemol (or chrysanthemic acid) were combined and the solvent evaporated carefully under reduced pressure in the cold. The purified compounds were characterized by optical rotation, NMR and IR spectroscopy, and mass spectrometry as follows

Product (+)-trans-chrysanthemic acid: yield 94 mg; optical rotation +11.8 (c=6.1 in ethanol); optical purity 83.4% based on lit. +14.16 (Merck Index, 10th Ed., p. 2231); IR (NaCl; $cm^{-1}$) 3000, 2950, 2920, 2895, 2840, 2640, 2530, 1675, 1425, 1363, 1335, 1310, 1270, 1228, 1200, 1170, 1135, 1098, 1070, 1052, 1002, 940, 840, 810, 690; 90MHz NMR ($CDCl_3$; ppm; multiplicity; assignment) 4.89 (1H, dq, J=1 and 7.8, olefinic-H), 2.18 (1H, dd, J=6 and 7.8, C3-H), 1.69 (6H, s, vinyl $CH_3$), 1.32, (1H, d, J=6, Cl-H), 1.28 and 1.12 (6H, 2s, geminal $CH_3$); EI-MS at 70 ev (m/z, % rel. abund.), M+ =168 (19.8), 153 (22.5), 135 (6), 123 (98.9), 107 (51.4), 95 (25.2), 91 (39.4), 81 (100).

Residual (-)-trans-chrysanthemol: yield 32 mg; optical rotation -39.2 (c=1.07 in cyclohexane); optical purity 78.9% based on lit for the (+)-isomer reported as +49.7 (Alexander and Epstein, J. Org. Chem., 40, 2576 (1975)); IR (NaCl; $cm^{-1}$) 3330, 2940, 2900, 2830, 2720, 1440, 1365, 1150, 1112, 1012, 980, 955, 842; 90 MHz ($CDCl_3$; ppm; multiplicity; assignment) 4.86 (1H, dq, J=1 and 7.8 Hz, olefinic-H), 3.65 (2H, AB center of ABX system, J=6 and 12 Hz, $-CH_2-OH$), 1.65 (6H, s, vinylic $CH_3$), 1.18 and 1.06 (6H, 2s, geminal $CH_3$); EI-MS at 70 ev (M/z, % rel. abund.), M+154 (9), 123 (100), 111 (18.2), 95 (10), 3 (12.1), 91 (9.5), 81 (67.8).

Product (+)-cis-chrysanthemic acid: yield 89 mg; optical rotation +31.2 (c=4.4 in ethanol); optical purity 78.6% based on lit. +39.69 (Matsui and Horichi, Agr. Biol. Chem., 35, 1984 (1971)); IR (NaCl; $cm^{-1}$) 3030, 2940, 2900, 2840, 2650, 2550, 1685, 1428, 1370, 1328, 1292, 1229, 1215, 1148, 1112, 1078, 1055, 995, 927, 840, 710; NMR ($CDCl_3$; ppm; multiplicity; assignment) 5.35 (1H, br d, J=7.5, olefinic-H), 1.92 (1H, t, J=7.5, C3-H), 1.70 and 1.68 (6H, 2s, vinylic $CH_3$), 1.20 and 1.17 (6H, 2S, geminal $CH_3$); EI-MS at 70 ev (identical to (+)-trans-chrysanthemic acid above).

Residual (-)-cis-chrysanthemol: yield 72 mg; optical rotation -33.4 (c=4.8 in dichloromethane); IR (NaCl; $cm^{-1}$) 3325, 2960, 2895, 2855, 2835, 2720, 1442, 1420, 1368, 1250, 1130, 1060, 1010, 838, NMR ($CDCl_3$; ppm; multiplicity assignment) 4.93 (1H, br d, J=7.8, olefinic-H), 3.6 (2H, d, J=6.9, $-CH_2-OH$), 1.65 (6H, s, olefinic $CH_3$), 1.32 (1H, t, J=7.6, C3-H), 1.08 and 0.98 (6H, 2s, geminal $CH_3$); EI-MS at 70 ev (identical to (-)-trans-chrysanthemol above).

9. Time Course Studies of the Microbial Oxidation of Chrysanthemols to the Corresponding Chrysanthemic Acids by Aspergillus ochraceus ATCC 18500 and A. flavipes ATCC 1030.

Study 1

A time course study was performed to determine whether there was a time-dependent diastereoselective oxidation of chrysanthemol substrates (i.e., oxidation of trans-stereoisomers prior to cis-stereoisomers, or vice versa).

Aspergillus ochraceus ATCC 18500 and A. flavipes ATCC 1030 were cultured in 125 ml first-stage flasks initiated by aseptically suspending surface growth or spores on agar slants in 25 ml of the following fermentation medium:

Bacto Soytone (Difco), 5 g;
yeast extract (Difco), 5 g;
NaCl, 5 g;
$K_2HPO_4$, 5 g;
microcrystalline cellulose, average particles size 10u (Brinkman), 5 g;
dextrose, 20 g;
distilled $H_20$, 1,000 ml;
pH adjusted to 6.0 with 6.0 N HCl

After incubation for 72 hrs, the entire contents of each first-stage flask was used to inoculate a one-liter second-stage flask of the same composition. The second-stage flasks were incubated for 48 hrs. 100 mg of chrysanthemol mixture, produced according to Section 3 above, in 0.4 ml of DMF was added to each flask. The first whole-flask samples of both fungal cultures were harvested 1 hr after the substrate addition and the initial transformation of chrysanthemols was then monitored by harvesting 2 ml culture aliquots from each flask every 6 hrs after the first detection of chrysanthemic acid formation, whole flasks were terminated at 10 hr intervals for Aspergillus ochraceus ATCC 18500 and 16 hr intervals for A. flavipes ATCC 1030.

20 ml of each culture was transferred to a 125 ml erlenmeyer flask which was then acidified with 10 ml Clarkes buffer, and a DMF solution of borneol was added (10 mg in 50 ul; i.e., the theoritical amount of total chrysanthemol-chrysanthemic acid mixture in 20 ml sample). Samples were homogenized and extracted with ether. Each ether extract was dried over anhydrous $MgSO_4$, filtered, and the solvent carefully evaporated to dryness in the cold under reduced pressure produced by a water aspirator. Following derivatization of each sample with diazoethane, as described in Section 4 above, samples were analyzed by GC as described in Section 5 above.

The microbial transformation of individual diastereomers of chrysanthemols by Aspergillus ochraceus ATCC 18500 is shown in Figure 3 and A. flavipes ATCC 1030 in Figure 4.

The study indicated that oxidation of the trans-alcohols begins first and is virtually completed before significant oxidation of the cis-alcohols begins. Oxidation of the trans-alcohols is completed at approximately 35 hrs and 50 hrs for A. ochraceus and A. flavipes, respectively. Oxidation of the cis -alcohols does not begin until approximately 30 hrs, and 50 hrs for A. ochraceus and A. flavipes, respectively. Since the trans-alcohols are oxidized before the cis-alcohols, the incubation can be interrupted and the trans-acid(s) can be extracted from the incubation mixture as described in Section 1 above. Incubation can then continue to produce cis-acid(s). As a result, the methods of the instant invention can be used to produce virtually stereochemically uniform cis-or trans-acids from racemic mixtures of alcohol substrate.

Study 2

(+/-)-cis, trans-chrysanthemols were employed as the substrate as described in Section 1 above, and samples were harvested at 24-hr intervals over 120 hrs. Samples were derivatized according to Section 4 above and were analyzed by GC as described in Section 5 above. Analysis of residual chrysanthemols and the products (chrysanthemic acids as their ethyl esters) clearly indicated that the trans-stereoisomers are metabolized prior to the cis-stereoisomers. Specifically, examination of A. flavipes ATCC 1030 at 24 and 48 hrs demonstrated that only trans-chrysanthemol oxidation had occurred, which was complete at 72 hrs (i.e., since this culture metabolizes both trans-chrysanthemol stereoisomers, no residual trans-alcohol remains). Cis-chrysanthemol oxidation was only evident at the 72 hr time interval and was virtually complete at 96 hrs (to the expected extent of 50% since this organism only oxidizes the (+)-cis-chrysanthemol).

With A. ochraceus ATCC 18500, 24 hr analysis indicated that oxidation of trans-chrysanthemol is complete (i.e., to the extent of 50% since only (+)-trans-chrysanthemol is oxidized) with no oxidation of the cis-stereoisomers. At 48 hrs, oxidation of cis-chrysanthemol is complete (i.e., to the extent of 50% since only (+)-cis-chrysanthemol is oxidized).

These data corroborate the findings on Study 1 above and indicate that racemic mixtures of alcohol substrates can be resolved into stereochemically pure cis-or trans-acids by the method of the instant invention.

Alternative Embodiments

The alcohol substrates (dibromochrysanthemol) and dichlorochrysanthemol) were obtained from decamethrin and permethrin by the following procedure. 5 g of decamethrin (or permethrin) was dissolved in 5% ethanolic NaOH (100 ml) and stirred overnight at room temperature under a nitrogen atmosphere. This solution was poured into 500 ml of an ice-water mixture and extracted with 200 ml hexane (×3) in a separatory funnel (this solvent extracts the nonacidic portion of the hydrolysate). The aqueous layer was carefully acidified with 6N HCl and extracted with ether (3×200 ml), and the ether phase was then dried over anhydrous $MgSO_4$, filtered and evaporated to dryness under reduced pressure. Yields were as follows: decamethric acid (2.4 g) and permethric acid (2.3 g). These acids were reduced with LAH in dry ether as described above to give dibromochrysanthemol (2.1 g) and permethrol (dichlorochrysanthemol) (2.1 g).

Microbial transformations of the dibromo and dichloro alcohols were carried out as described above with A. ochraceus (ATCC 18500) and A. flavipes (ATCC 1030) by culture screening using two-125 ml second stage flasks for each culture. Samples were analyzed by gas chromatography as their ethyl esters described above. The dibromo and dichloro alcohols were transformed to their corresponding acids as shown in Figures 5-8.

Microbial transformation of a racemic mixture of lavandulol was performed as follows. Six one-liter second-stage growing cultures of Aspergillus ochraceus (ATCC 18500) were generated as described in Study 1 of Section 9 above. After 36 hours incubation, the substrate, RS-lavandulol was added to a level of 80 mg per flask (in 40 ul DMF). The incubation was terminated when chromatographic analysis indicated an approximate 50% conversion. The culture was acidified and exhaustively extracted with ether, and the crude extract subjected to column chromatography on Sephadex LH-20 eluted with cyclohexane:dichloromethane:ethanol (7:4:1). Those fractions representing nearly pure product, lavandulic acid, were combined with partitioned between 5% aqueous sodium bicarbonate solution and pentane to remove traces of unmetabolized substrate, lavandulol. The isolated acid (37 mg) was homogenous by thin-layer chromatography, and yielded an optical rotation indicative of substantial enrichment in the (-)-enantiomer of lavandulic acid, $[\alpha]^{22}D = -22.8$ (c = 3.7, chloroform).

## Claims

1. A method for the production of an acid of the formula

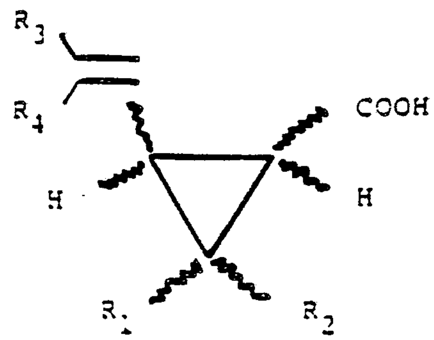

I

comprising adding together and incubating under oxidizing conditions (1) an alcohol substrate of the formula

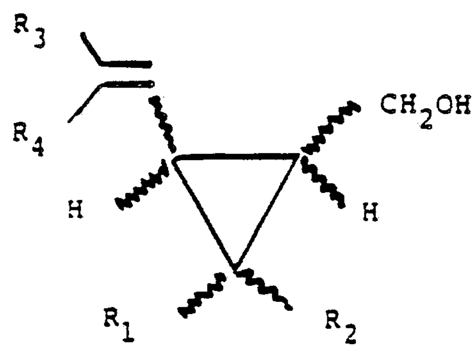

II

and (2) one or more oxidizing enzymes originated from a microorganism, wherein

$R_1$ is selected from the group consisting of a hydrogen atom, $-CH_3$, $-CH_2CH_3$, $-CH_2CH_2CH_3$, and a halogen atom;

$R_2$ is selected from the group consisting of a hydrogen atom, $-CH_3$, $-CH_2CH_3$, $-CH_2CH_2CH_3$, and a halogen atom;

$R_3$ is selected from the group consisting of $-CH_3$, $-CH_2CH_3$, $-CH_2CH_2CH_3$, $-CH_2CH_2CH_2CH_3$, $-CH_2CH_2CH_2CH_2CH_3$, and a halogen atom; and

$R_4$ is selected from the group consisting of $-CH_3$, $CH_2CH_3$, $-CH_2CH_2CH_3$, $-CH_2CH_2CH_2CH_3$, $-CH_2CH_2CH_2CH_2CH_3$, and a halogen atom, and $COOCH_3$.

2. The method of claim 1 wherein

the alcohol substrate comprises one or more stereoisomers selected from the group consisting or (1R,3R) (+)-trans stereoisomer, (1S,3S) (-)-trans stereoisomer, (1S,3R) (+)-cis stereoisomer and (1R,3S) (-)-cis stereoisomer; and

the acid comprises a stereoisomer which is stereochemically analogous to one of the one or more of the alcohol substrate's stereoisomers such that at least one of the one or more of the alcohol substrate's stereoisomers is transformed to the stereochemically analogous stereoisomer of the acid.

3. The method of claim 1 or 2 wherein

$R_1$ and $R_2$ are $-CH_3$;

$R_3$ is selected from the group consisting of $-CH_3$, a chlorine atom and a bromine atom; and

$R_4$ is identical to $R_3$.

4. The method of claim 2 wherein the alcohol substrate comprises at least two of said alcohol substrate's stereoisomers and the acid comprises one or more stereoisomers which are stereochemically analogous respectively to one or more of the at least two of the alcohol's stereoisomers such that one or more of the at least two of the alcohol substrate's stereoisomers are transformed to the stereochemically analogous stereoisomer of the acid.

5. The method of claim 4 wherein the acid comprises stereoisomers which are stereochemically analogous to some of but not all of the stereoisomers of the alcohol substrate such that only some of the stereoisomers of the alcohol substrate are transformed to the stereochemically analogous stereoisomers of the acid in amounts in excess of trace amounts.

6. The method of claim 5 wherein $R_1$, $R_2$, $R_3$ and $R_4$ are $-CH_3$ and
the alcohol substrate comprises the (1R,3R) (+)-trans stereoisomer, the (1S,3S) (-)-trans stereoisomer, the (1S,3R) (-)-cis stereoisomer and the (1R,3S) (+)-cis stereoisomer; and
the acid comprises the (1R,3R) (+)-trans stereoisomer and the (1R,3S) (+)-cis stereoisomer with at most trace amounts of the (1S,3S) (-)-trans stereoisomer and the (1S,3R) (-)-cis stereoisomer.

7. The method of claim 5 wherein the one or more enzymes transforms to the corresponding acid substantial portions of (1R,3R) (+)-trans, if any, stereoisomer and (1R,3S) (+)-cis stereoisomer, if any, contained in the alcohol substrate but does not transform beyond at most amounts to the corresponding acid (1S,3S) (-)-trans stereoisomer, if any, or (1S,3R) (-) stereoisomer, if any, contained in the alcohol substrate.

8. The method of claim 5 wherein $R_1$, $R_2$, $R_3$ and $R_3$ are $-CH_3$ and
the alcohol substrate comprises the (1R,3R) (+)-trans stereoisomer, the (1S,3S) (-)-trans stereoisomer, the (1S,3R) (-)-cis stereoisomer and the (1R,3S) (+)-cis stereoisomer; and
the acid comprises the (1R,3R) (+)-trans stereoisomer and the (1S,3S) (-)-trans stereoisomer with at most trace amounts of the (1S,3R) (-)-cis stereoisomer and the (1R,3S) (+)-cis stereoisomer.

9. The method of claim 5 wherein the one or more enzymes transforms to the corresponding acid substantial portions of (1R,3R) (+)-trans stereoisomer, if any, and (1S,3S) (-)-trans stereoisomer, if any, contained in the alcohol substrate but does not transform beyond at most trace amounts to the corresponding acid (1S,3R) (-)-cis stereoisomer, if any, or (1R,3S) (+)-cis stereoisomer, if any, contained in the alcohol substrate.

10. The method of claim 9 wherein $R_1$, $R_2$, $R_3$ and $R_4$ are $-CH_3$ and the microorganism is Aspergillus flavipes (ATCC 1030).

11. The method of claim 10 wherein said incubation is interrupted after about 45 hours to about 55 hours, preferably after about 65 hours to about 75 hours.

12. The method of any of claims 9 to 11 further comprising
extracting said (1R,3R) (+)-trans stereoisomer, if any, and said (1S,3S) (-)-trans-stereoisomer, if any, of said acid; and
continuing said incubation after said extraction so as to produce acid comprising substantial portions of said (1S,3R) (-)-cis-stereoisomer, if any, and said (1R,3S) (+)-cis-stereoisomer, if any.

13. The method of claim 9 wherein $R_1$, $R_2$, $R_3$ and $R_4$ are $-CH_3$ and
the alcohol substrate comprises the (1R,3R) (+)-trans-stereoisomer; and
the acid comprises the (1R,3R) (+)-trans-stereoisomer with at most trace amounts of the (1R,3S) (+)-cis-stereoisomer.

14. The method of claim 13 wherein the microorganism is Aspergillus ochraceus (ATCC 18500).

15. The method of claim 14 wherein said incubation is interrupted after about 20 hours to about 30 hours, preferably after about 30 hours to about 40 hours.

16. The method of any of the claims 13 to 15 further comprising
extracting said (1R,3R) (+)-trans-stereoisomer of said acid; and
continuing said incubation after said extraction so as to produce acid comprising a substantial portion of said (1R,3S) (+)-cis-stereoisomer.

17. The method of claim 5 wherein $R_1$, $R_2$, $R_3$ and $R_4$ are $-CH_3$ and
the alcohol substrate comprises the (1S,3R) (-)-cis stereoisomer and the (1R,3S) (+)-cis stereoisomer; and
the acid comprises the (1R,3S) (+)-cis stereoisomer with at most trace amounts of the (1S,3R) (-)-cis stereoisomer.

18. The method of claim 17 wherein the (1R,3S) (+)-cis stereoisomer of the acid is not produced in amounts greater than trace amounts until substantially all of the (1R,3R) (+)-trans stereoisomer, if any, and the (1S,3S) (-)-trans stereoisomer, if any, contained in the alcohol substrate has already been transformed by the one or more enzymes.

19. A method for the production of an acid of the formula

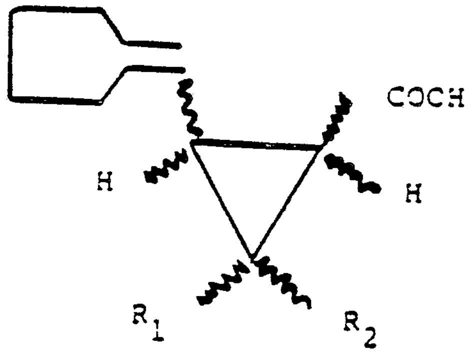

III

comprising adding together and incubating under oxidizing conditions (1) an alcohol substrate of the formula

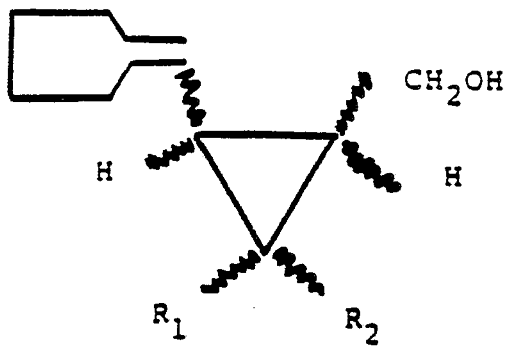

IV

and (2) one or more oxidizing enzymes originated from a microorganism, wherein

$R_1$ is selected from the group consisting of a hydrogen atom, $-CH_3$, $-CH_2CH_3$, $-CH_2CH_2CH_3$, and a halogen atom; and

$R_2$ is selected from the group consisting of a hydrogen atom, $-CH_3$, $-CH_2CH_3$, $-CH_2CH_2CH_3$, and a halogen atom.

20. The method of claim 19 wherein

the alcohol substrate comprises one or more stereoisomers selected from the group consisting of (1R,3R) (+)-trans stereoisomer, (1S,3S) (-)-trans stereoisomer, (1R,3S) (+)-cis stereoisomer and (1S,3R) (-)-cis stereoisomer; and

the acid comprises a stereoisomer which is stereochemically analogous to one of the one or more of the alcohol substrate's stereoisomers such that at least one of the one or more of the alcohol substrate's stereoisomers is transformed to the stereochemically analogous stereoisomer of the acid.

21. The method of claim 19 or 20 wherein

$R_1$ and $R_2$ are $-CH_3$.

22. The method of any of the claims 1, 2, 19, 20, wherein the microorganism is selected from Aspergillus ochraceus (ATCC 18500), Aspergillus flavipes (ATCC 1030) and Aspergillus flavipes (ATCC 11013).

23. The method of any of the claims 1, 2, 19, 20, wherein the microorganism is transformed to contain genes for said one or more enzymes.

24. The method of any of the claims 1 to 23, wherein the microorganism or the one or more enzymes are immobilized during said incubation.

25. A method for the production of an acid of the formula

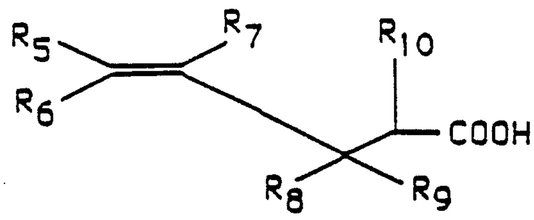

V

comprising adding together and incubating under oxidizing conditions (1) an alcohol substrate of the formula

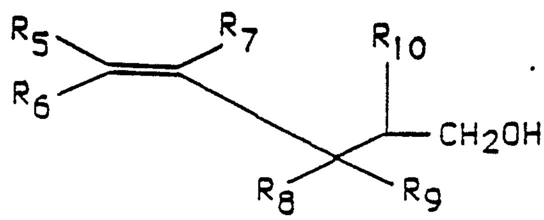

VI

and (2) one or more oxidizing enzymes originated from a microorganism, wherein

$R_5$ is selected from the group consisting of $-CH_3$, a hydrogen atom and a halogen atom;

$R_6$ is selected from the group consisting of a $-CH_3$, hydrogen atom and a halogen atom;

$R_7$ is selected from the group consisting of a hydrogen atom, a halogen atom and $-CH_3$;

$R_8$ is selected from the group consisting of a hydrogen atom, $-CH_3$ and $-CH_2CH_3$;

$R_9$ is selected from the group consisting of a hydrogen atom and $-CH_3$; and

$R_{10}$ is selected from the group consisting of $-CH_3$, $-CH_2CH_3$, an isopropylene group an isopropyl group, a sec-butyl group, an isobutyl group, a t-butyl group, a cyclopentyl group, a cylopropyl group.

26. The method of claim 25 wherein

the alcohol substrate comprises one or more stereoisomers selected from the group consisting of R-(-) stereoisomer and S-(+) stereoisomer; and

the acid comprises a stereoisomer which is stereochemically analagous to one of the one or more of the alcohol substrate's stereoisomers such that at least one of the one or more of the alcohol substrate's stereoisomers is transformed to the stereochemically analogous stereoisomer of the acid.

27. The method of claim 26 wherein

$R_5$ and $R_6$ are $-CH_3$;

$R_7$, $R_8$ and $R_9$ are a hydrogen atom; and

$R_{10}$ is an isopropylene group.

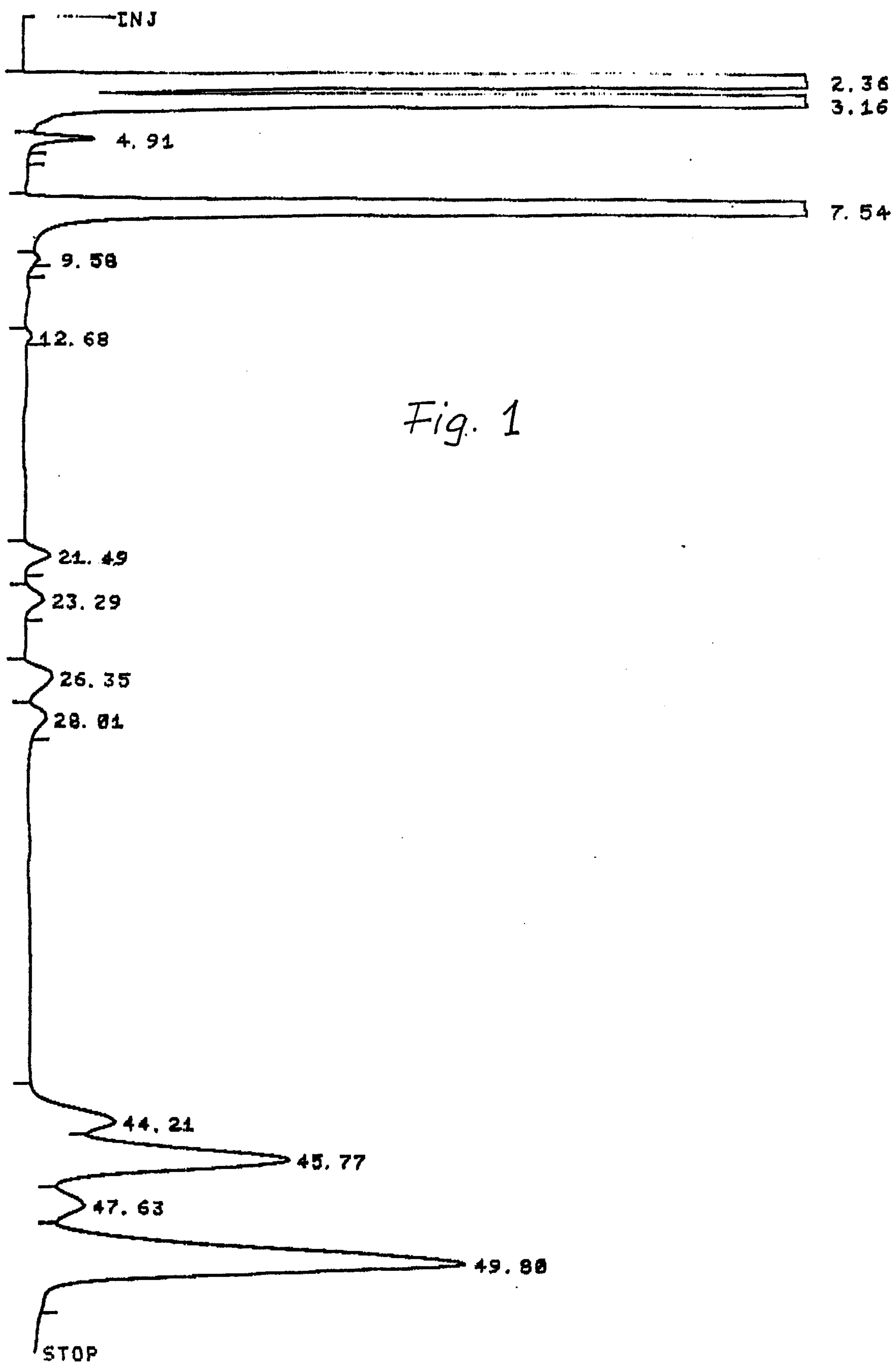
INJ
2.36
3.16
4.91
7.54
9.58
12.68
21.49
23.29
26.35
28.01
44.21
45.77
47.63
49.80
STOP

Fig. 1

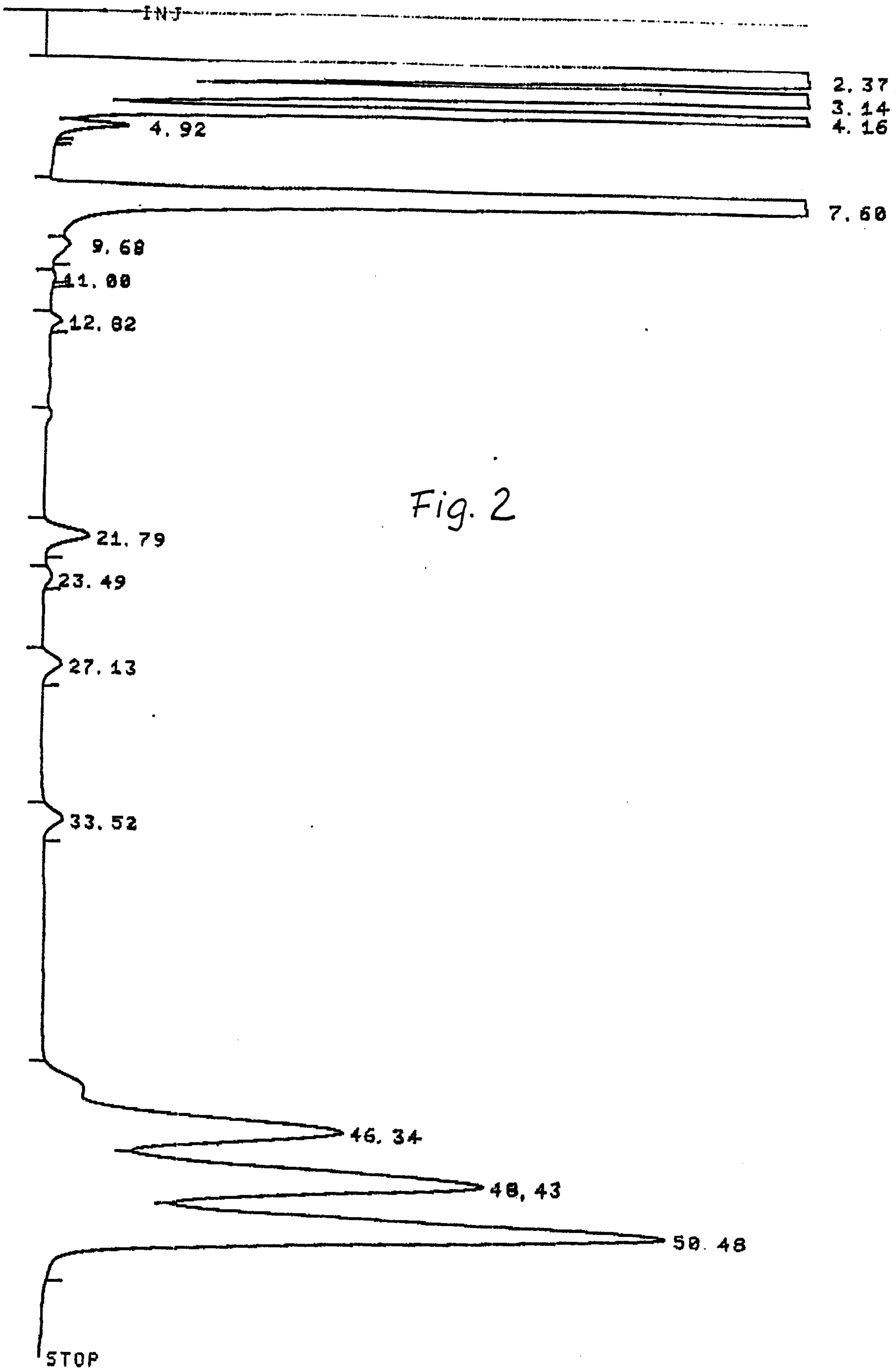
INJ
2.37
3.14
4.16
4.92
7.60
9.68
11.00
12.82
21.79
23.49
27.13
33.52
46.34
48.43
50.48
STOP

Fig. 2

FIGURE 3. DIASTEREOSELECTIVE TIME COURSE OF CHRYSANTHEMYL ALCOHOL OXIDATION BY ASPERGILLUS OCHRACEUS (ATCC 18500)

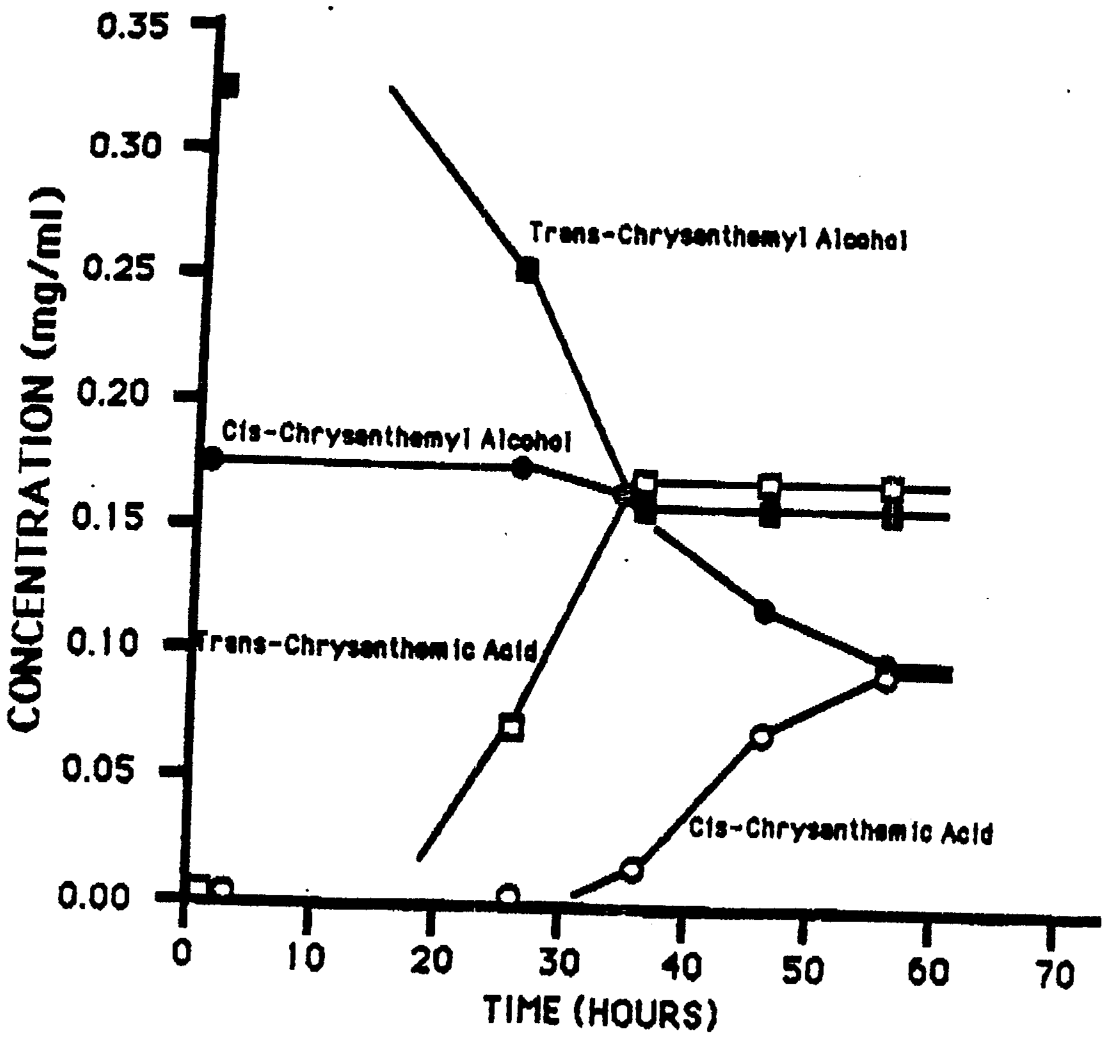

FIGURE 4. DIASTEREOSELECTIVE TIME COURSE OF CHRYSANTHEMYL ALCOHOL OXIDATION BY ASPERGULLUS FLAVIPES (ATCC 1030)

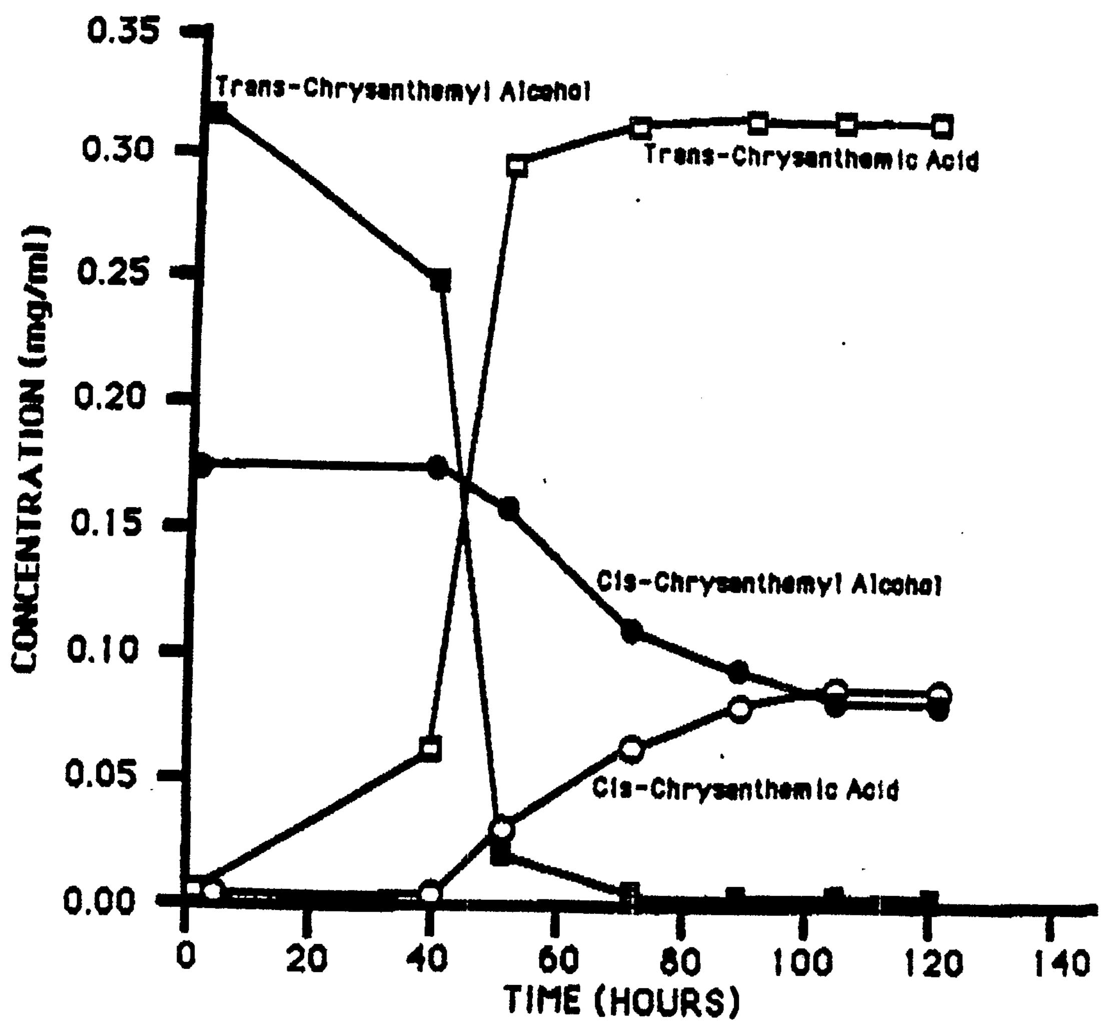

Fig. 5

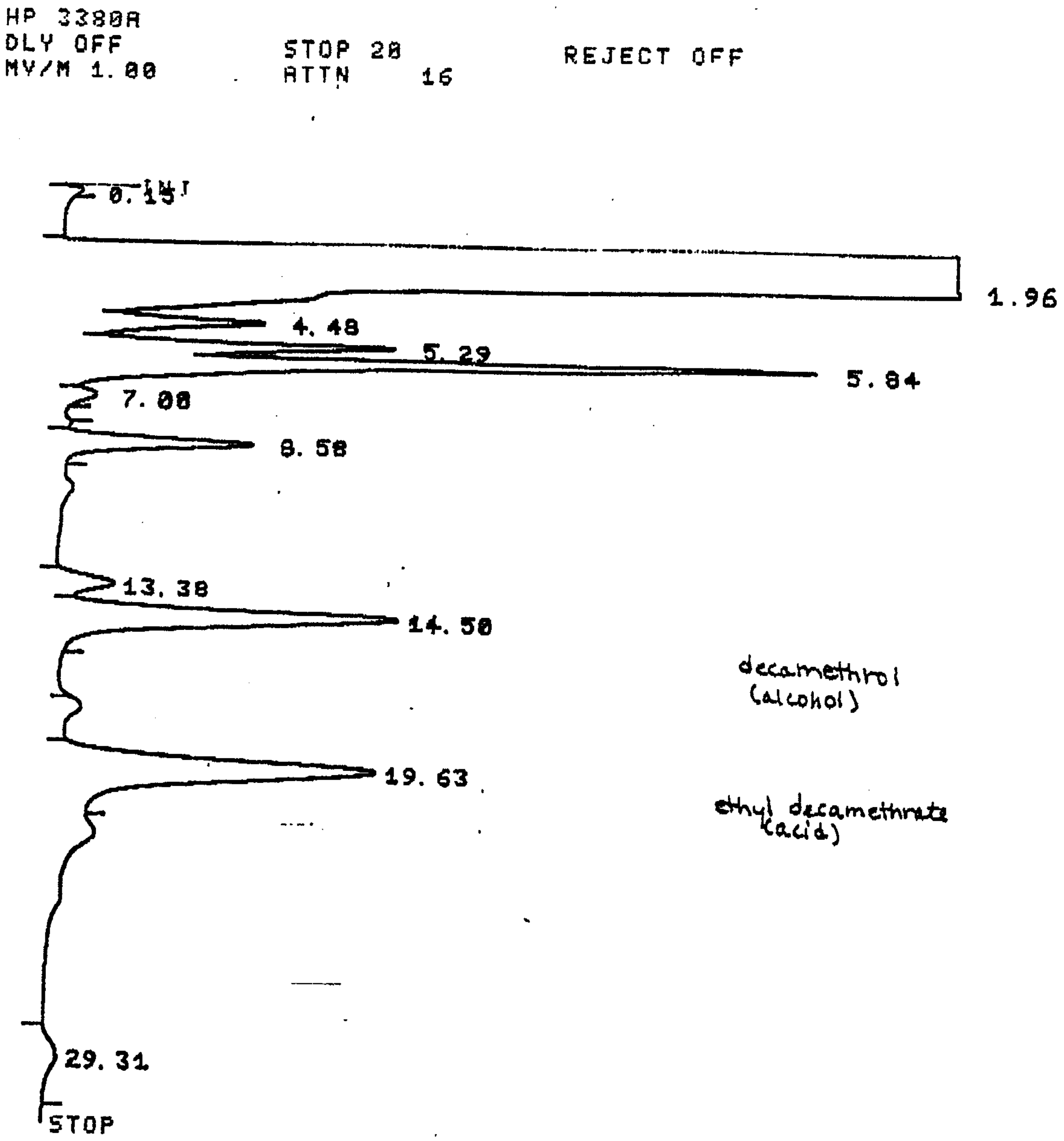
HP 3380A
DLY OFF
MV/M 1.00
STOP 20
ATTN 16
REJECT OFF
INJ
0.15
1.96
4.48
5.29
5.84
7.00
8.58
13.38
14.50
decamethrol
(alcohol)
19.63
ethyl decamethrate
(acid)
29.31
STOP

Fig. 6

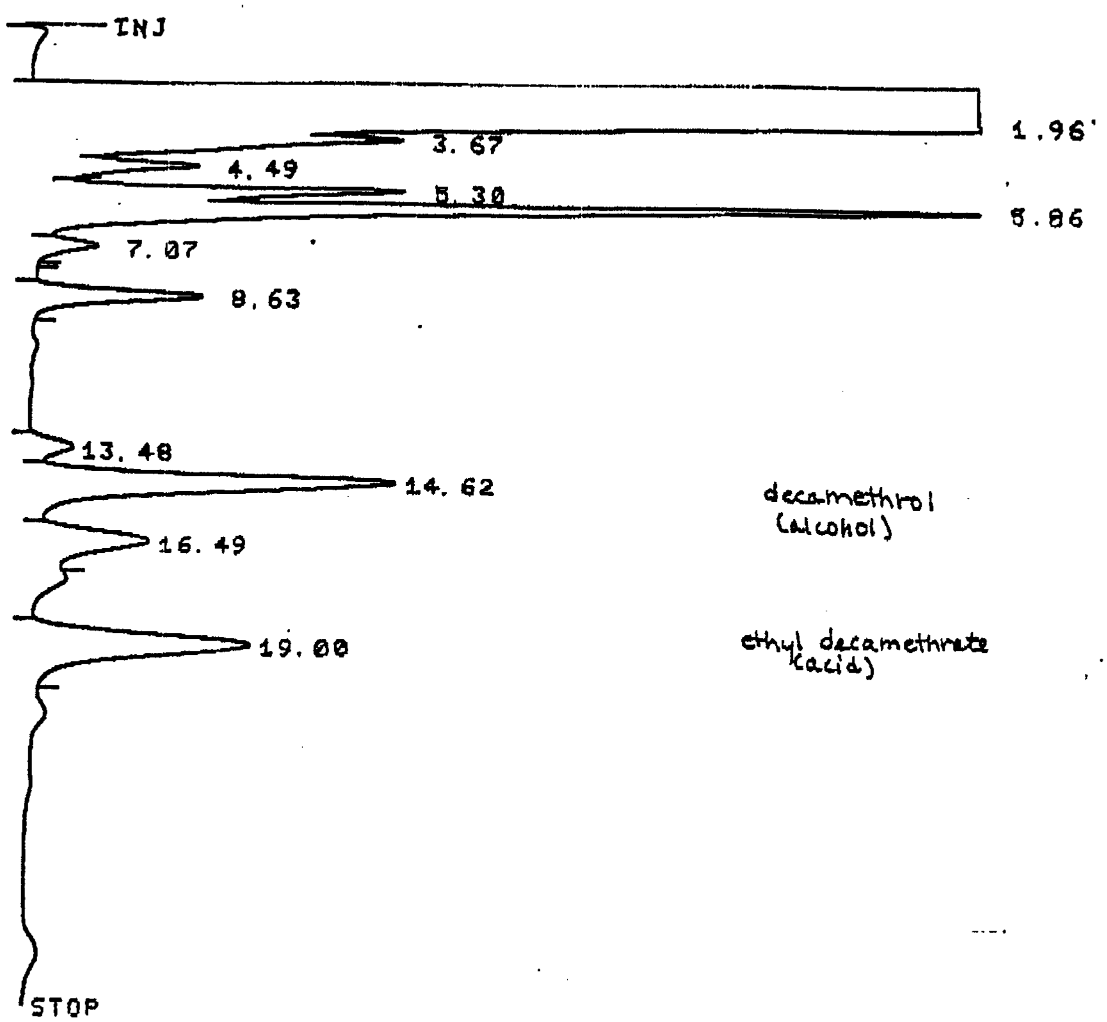
INJ
1.96
3.67
4.49
5.30
5.86
7.07
8.63
13.48
14.62
16.49
19.00
STOP
decamethrol
(alcohol)
ethyl decamethrate
(acid)

Fig. 7

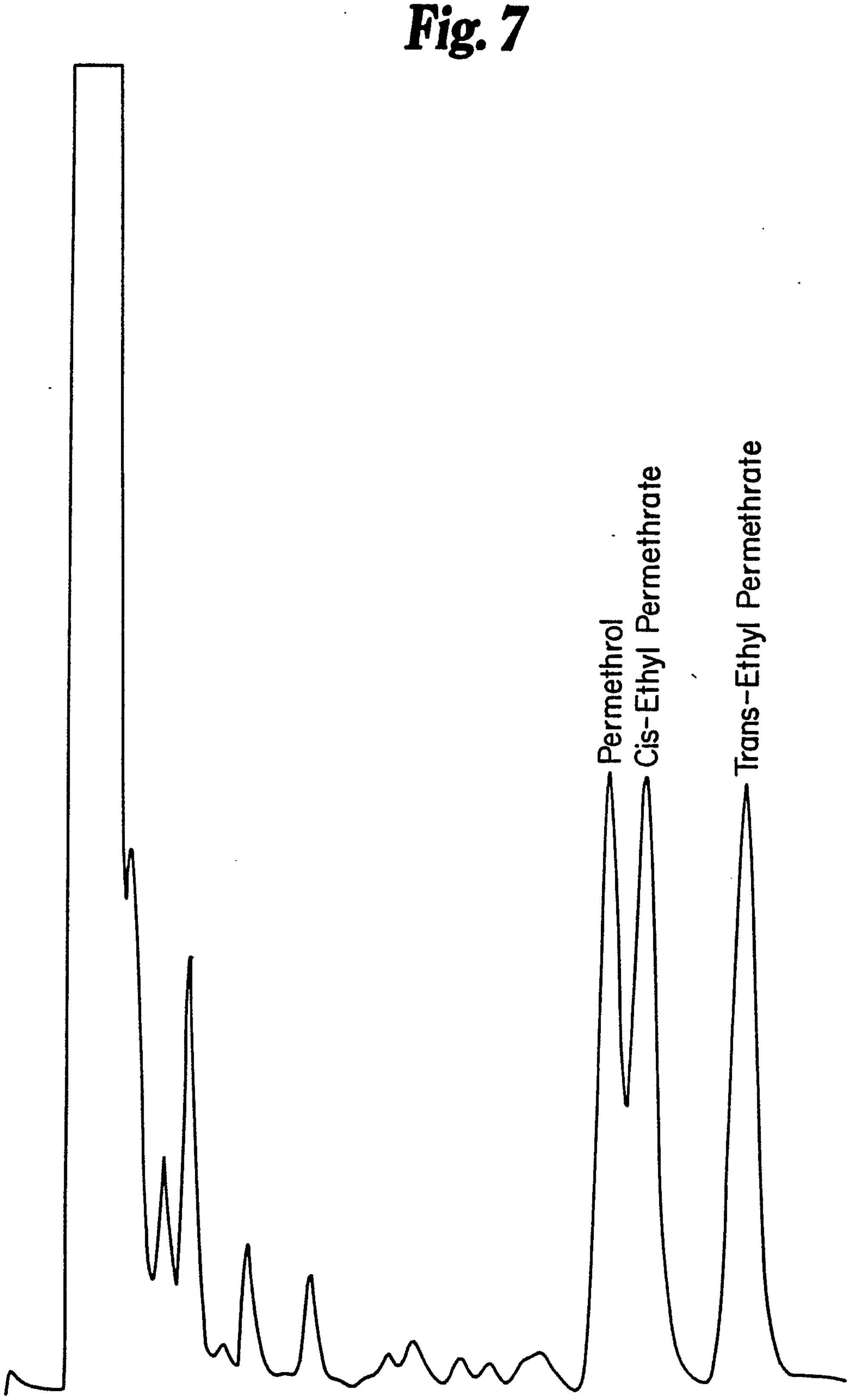
Permethrol
Cis-Ethyl Permethrate
Trans-Ethyl Permethrate

Fig. 8

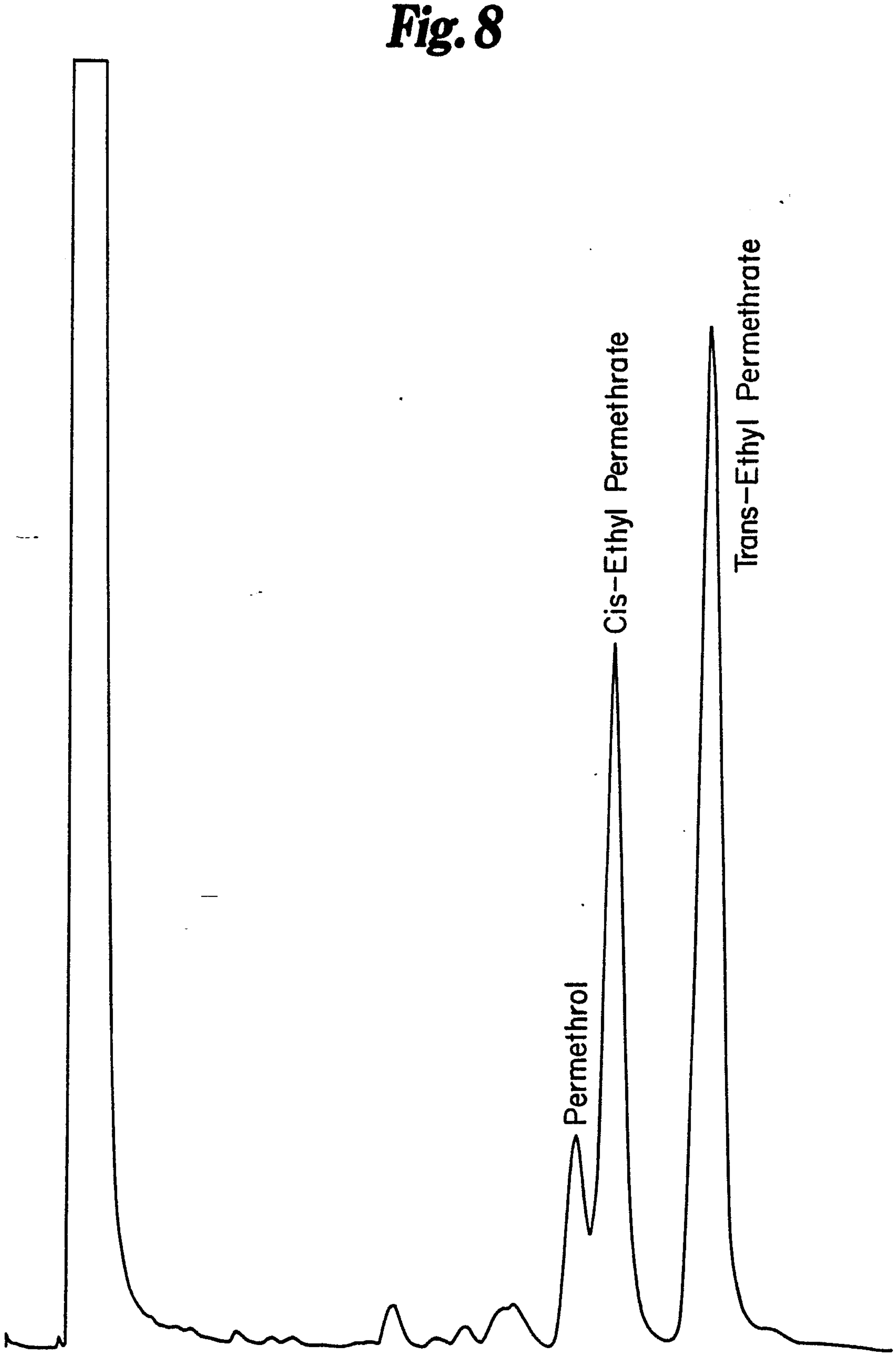
Permethrol
Cis-Ethyl Permethrate
Trans-Ethyl Permethrate